# EUROPEAN PATENT SPECIFICATION

(11) **EP 2 704 768 B1**
(45) Date of publication and mention of the grant of the patent: **10.10.2018**
(21) Application number: 12718673.2
(22) Date of filing: 04.05.2012
(51) Int. Cl.: A61M 5/19, A61M 5/20, A61M 5/24

(54) **REUSE PROTECTION FOR DISPOSABLE PARTS**
WIEDERVERWENDBARER SCHUTZ FÜR EINWEGTEILE
PROTECTION CONTRE LA RÉUTILISATION DE PIÈCES JETABLES

(30) Priority: 06.05.2011 EP 11165115
(43) Date of publication of application: 12.03.2014
(73) Proprietor: Sanofi-Aventis Deutschland GmbH, 65929 Frankfurt am Main (DE)
(72) Inventor: NESSEL, Christian, 65926 Frankfurt am Main (DE)
(74) Representative: McDougall, James
(86) International application number: PCT/EP2012/058254
(87) International publication number: WO 2012/152694

(56) References cited:
- WO-A1-03/103757
- WO-A2-03/051423
- WO-A2-2007/101798
- US-A- 5 833 660

## Description

The present patent application inter-alia relates to medical devices for ejecting a dose of a medicament. For instance, the present patent application relates to medical devices of delivering at least two drug agents from separate reservoirs. Such drug agents may comprise a first and a second medicament. The medical device includes a dose setting mechanism for delivering the drug automatically or manually by the user.

The drug agents may be contained in two or more multiple dose reservoirs, containers or packages, each containing independent (single drug compound) or pre-mixed (co-formulated multiple drug compounds) drug agents.

Certain disease states require treatment using one or more different medicaments. Some drug compounds need to be delivered in a specific relationship with each other in order to deliver the optimum therapeutic dose. The present patent application is of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin (also may be referred to as the first or primary medicament) along with a glucagon-like peptide-1 such as GLP-1 or GLP-1 analog (also may be referred to as the second drug or secondary medicament).

Accordingly, there exists a need to provide devices for the delivery of two or more medicaments in a single injection or delivery step that is simple for the user to perform without complicated physical manipulations of the drug delivery device. The proposed drug delivery device provides separate storage containers or cartridge retainers for two or more active drug agents. These active drug agents are then only combined and/or delivered to the patient during a single delivery procedure. These active agents may be administered together in a combined dose or alternatively, these active agents may be combined in a sequential manner, one after the other.

The drug delivery device also allows for the opportunity of varying the quantity of the medicaments. For example, one fluid quantity can be varied by changing the properties of the injection device (e.g., setting a user variable dose or changing the device's "fixed" dose). The second medicament quantity can be changed by manufacturing a variety of secondary drug containing packages with each variant containing a different volume and/or concentration of the second active agent.

The drug delivery device may have a single dispense interface. This interface may be configured for fluid communication with the primary reservoir and with a secondary reservoir of medicament containing at least one drug agent. The drug dispense interface can be a type of outlet that allows the two or more medicaments to exit the system and be delivered to the patient.

The combination of compounds as discrete units or as a mixed unit can be delivered to the body via a double-ended needle assembly. This would provide a combination drug injection system that, from a user's perspective, would be achieved in a manner that closely matches the currently available injection devices that use standard needle assemblies. One possible delivery procedure may involve the following steps:
1. Attach a dispense interface to a distal end of the electro-mechanical injection device. The dispense interface comprises a first and a second proximal needle. The first and second needles pierce a first reservoir containing a primary compound and a second reservoir containing a secondary compound, respectively.
2. Attach a dose dispenser, such as a double-ended needle assembly, to a distal end of the dispense interface. In this manner, a proximal end of the needle assembly is in fluidic communication with both the primary compound and secondary compound.
3. Dial up/set a desired dose of the primary compound from the injection device, for example, via a graphical user interface (GUI).
4. After the user sets the dose of the primary compound, the micro-processor controlled control unit may determine or compute a dose of the secondary compound and preferably may determine or compute this second dose based on a previously stored therapeutic dose profile. It is this computed combination of medicaments that will then be injected by the user. The therapeutic dose profile may be user selectable.
5. Optionally, after the second dose has been computed, the device may be placed in an armed condition. In such an optional armed condition, this may be achieved by pressing and/or holding an "OK" button on a control panel. This condition may provide for greater than a predefined period of time before the device can be used to dispense the combined dose.
6. Then, the user will insert or apply the distal end of the dose dispenser (e.g., a double ended needle assembly) into the desired injection site. The dose of the combination of the primary compound and the secondary compound (and potentially a third medicament) is administered by activating an injection user interface (e.g., an injection button).

Both medicaments may be delivered via one injection needle or dose dispenser and in one injection step. This offers a convenient benefit to the user in terms of reduced user steps compared to administering two separate injections.

After a specific number of injections (e.g. 1 injection, 3 injections, 5 injections, 10 injections, 20 injections, 50 injections or the like) there is a risk for the dose dispenser and/or the dispense interface to be contaminated and, additionally, the tips of the needles of the dose dispenser and/or the dispense interface may be blunted. For instance, a blunted tip of a needle may not be able to sufficiently pierce a septum and/or tissue, for instance inserting a blunted needle in a desired injection site may be very painful. Furthermore, mechanical parts of the dose dispenser and/or the dispense interface such as a valve arrangement may only proper function for a specific number of injections (e.g. 1 injection, 3 injections, 5 injections, 10 injections, 20 injections, 50 injections or the like).

Therefore, the dose dispenser and the dispense interface are disposable parts. For instance, the dose dispenser should only be used for one injection and the dispense interface should only be used with one first and second reservoir. However, users may, for instance accidentally or in order to save costs, reuse disposable parts such as the dose dispenser and the dispense interface.

Therefore, the present invention inter-alia faces the technical problem of providing a reuse protection for disposable parts such as a dose dispenser and a dispense interface.

A reuse protector is known from WO 03/103757 A1, preventing the reuse of a needle tip by locking the needle after use with a needle guard.

In accordance with a first aspect of the present invention, there is provided a dispense interface attachable to a medical device, the dispense interface comprising a receiving opening comprising blades arranged to form an opening and to be constricted in the direction of the centre of the opening by an elastic force, the receiving opening having a closed condition and a spread condition, wherein in the closed condition the opening has an opening diameter that is smaller than an outer diameter of a connecting part of the medical device, and in the spread condition the opening diameter of the opening is at least as big as the outer diameter of the connecting part of the medical device, and the receiving opening is configured to receive the connecting part of the medical device when in the spread condition and to block receiving said connecting part of said medical device when in the closed condition; and a spreader that is at least partially tapered and insertable into the receiving opening and the spreader is configured, during insertion, to move into the receiving opening such that the blades slip along the at least partially tapered surface of the spreader to spread the blades of said receiving opening and enlarge an opening diameter of the receiving opening, when the receiving opening is in the spread condition.

Furthermore, in accordance with a second aspect of the present invention, there is provided a method of reuse protection for a dispense interface, the method comprising: spreading, by the spreader, the blades of the receiving opening when the receiving opening is in the spread condition, such that the opening diameter of the opening is at least as big as the outer diameter of the connecting part of the medical device; receiving, when the receiving opening is in the spread condition, in said receiving opening, the connecting part of the medical device; moving, when receiving said connecting part of said medical device, said spreader from a first position in said receiving opening to a second position in said receiving opening; locking, when the spreader is in the second position, said spreader at the base of the receiving opening; and blocking, when the receiving opening is in the closed condition, said receiving said connecting part of said medical device.

The medical device may be drug delivery device such as a medical device configured to eject a drug agent (e.g. a dose of a medicament) such as an infusion device or an injection device, for instance an insulin injection pen. Injection devices may be used either by medical personnel or by patients themselves. As an example, type-1 and type-2 diabetes may be treated by patients themselves by injection of insulin doses, for example once or several times per day.

For instance, the medical device is configured to eject at least two drug agents from separate reservoirs comprising a first and a second medicament, respectively, but it is not limited thereto. Alternatively, the medical device is for instance a conventional medical device configured to eject a drug agent from a single reservoir such as Applicant's Solostar insulin injection pen.

The dispense interface may be a disposable part attachable to the medical device. For instance, the dispense interface may be attachable to the medical device by use of mating attachment means (e.g. axial attachment means) forming a frictional fit, a form fit and/or an interference fit. Examples of mating attachment means include snap locks, snap fits, snap rings, keyed slots, threads and any combinations thereof. Furthermore, the dispense interface may only be attachable to specific types of medical devices.

A dispense interface may be configured to be in fluid communication with at least one reservoir of the medical device containing at least one medicament. For instance, the drug dispense interface is a type of outlet that allows the at least one medicament to exit the medical device. The dispense interface may comprise a valve arrangement. Such a valve arrangement may be configured to prevent contamination of the at least one medicament in the at least one reservoir. For instance, the valve arrangement is configured to prevent back flow of the at least one medicament when the medicament is delivered/ejected.

A dose dispenser may be configured to be in fluid communication with at least one reservoir of the medical device containing at least one medicament and/or with a dispense interface. For instance, the dose dispenser is a needle assembly, for instance a double-ended needle assembly and/or a standard needle assembly. One end of such a needle assembly may be inserted into a desired injection site before an injection.

The receiving opening may be an opening in which a connecting part of the medical device is to be received to enable an attachment of the dispense interface to the medical device. The connecting part of the medical device may at least partially to be received in the receiving opening in order to enable a cooperation of the mating attachment means of the dispense interface and the medical device. For instance, the mating attachment means of the dispense interface and the medical device may be aligned when the connecting part is at least partially received in the receiving opening.

In particular, the connecting part of the medical device may be a connecting part of at least one reservoir of the medical device. The reservoir is for instance a replaceable cartridge and/or a refillable container containing a medicament. For instance, the connecting part of the medical device is configured to enable a fluid communication between the at least one reservoir of the medical device and the dispense interface. The connecting part of the medical device may comprise a fluid connector such as a valve arrangement, a piercable septum or the like.

The receiving opening may at least partially be spreadable such that at least the opening diameter of the receiving opening is enlargeable by spreading the receiving opening. For instance, the receiving opening is made from an elastic material. The diameter of the receiving opening may not be constant. For instance, the opening diameter of the receiving opening may be the minimum (cross-sectional) diameter of the receiving opening.

In the spread condition, the opening diameter of the receiving opening is for instance enlarged such that the opening diameter of the receiving opening is at least as big as an outer diameter of the connecting part of the medical device. The outer diameter of the connecting part may be the maximum (cross-sectional) diameter of the connecting part of the medical device.

In the relaxed condition, the opening diameter of the receiving opening is for instance not enlarged or not sufficiently enlarged such that the opening diameter of the receiving opening is smaller than the outer diameter of the connecting part of the medical device. In the relaxed condition, the receiving opening may be closed.

The receiving opening may have the shape of a (hollow) truncated cone, wherein the truncated end of the truncated cone-shaped receiving opening may be open. The diameter of the base of this truncated cone-shaped receiving opening may be at least as big as the outer diameter of the connecting part of the medical device; and the diameter of the open, truncated end of the truncated cone-shaped receiving opening may be the opening diameter of the receiving opening.

The spreader may be at least partially insertable in the receiving opening such that the receiving opening is spread (e.g. the opening diameter of the receiving opening is enlarged and is as big as the outer diameter of the spreader). The spreader may be arranged in the open, truncated end of the truncated cone-shaped receiving opening. For instance, the outer diameter of the spreader is at least as big as the outer diameter of the connecting part. Thus, when the spreader is inserted in the receiving opening as described above, the receiving opening may be in the spread condition. When the spreader is for instance removed from the receiving opening, the receiving opening may be in the relaxed condition. Accordingly, inserting and removing the spreader may allow changing the condition of the receiving opening.

In the spread condition, the connecting part of the medical device is for instance at least partially receivable in the receiving opening such that a cooperation of the mating attachment means of the dispense interface and the medical device is enabled. In contrast to this in the relaxed condition, the connecting part of the medical device is for instance not or only partially receivable in the receiving opening such that a cooperation of the mating attachment means of the dispense interface and the medical device is disabled. For instance, the attachment means of the dispense interface and the medical device are only aligned, if the connecting part is entirely or partially received in the receiving opening.

The spread and relaxed condition may inter-alia enable and disable, respectively, receiving a connecting part of the medical device in the receiving opening and/or an attachment of the dispense interface to the medical device. By changing the condition of the receiving opening from the initial spread condition to the relaxed condition, after the dispense interface is attached to the medical device, a reuse of the dispense interface may be prevented. This is inter-alia advantageous in order to prevent a reuse of the dispense interface.

In the following, features and embodiments (exhibiting further features) of the present invention will be described, which are understood to equally apply to the dispense interface and the method as described above. These single features/embodiments are considered to be exemplary and non-limiting, and to be respectively combinable independently from other disclosed features/embodiments of the dispense interface and the method as described above. Nevertheless, these features/embodiments shall also be considered to be disclosed in all possible combinations with each other and with the dispense interface and the method as described above. For instance, a mentioning that a dispense interface according to the present invention is configured to perform a certain action should be understood to also disclose an according method step of the method according to the present invention.

According to an embodiment, the spreader is further configured to relax the receiving opening in the relaxed condition such that the opening diameter of the receiving opening is smaller than the outer diameter of the connecting part of the medical device. For instance, if the spreader is at least partially removed from the receiving opening, the receiving opening may at least partially relax such that the opening diameter of the receiving opening is in the relaxed condition.

This embodiment is inter-alia advantageous in order to allow changing the condition of the receiving opening.

According to an embodiment, the dispense interface further comprises a connector configured to connect to the connecting part of the medical device, wherein the connector is arranged in the receiving opening.

The connector may be configured to enable a fluid communication between the dispense interface and the medical device. For instance, the connector is configured to enable a fluid communication with a reservoir of the medical device such as a cartridge. In particular, the connector of the dispense interface may correspond to a connector of the connecting part of the medical device. These mating connectors may be any known fluid connectors. For instance, the mating connectors may be mating luer-taper connectors. Alternatively, the connector of the dispense interface may be a needle configured for piercing a septum of the medical device, for instance a septum of a reservoir such as a cartridge and/or a container.

The connector of the dispense interface is arranged in the receiving opening. In particular, the connector of the dispense interface may be arranged in the receiving opening such that the connector is only connectable with the connecting part of the medical device, if the connecting part of the medical device is at least partially receivable in the receiving opening. For instance, the receiving opening encompasses the connector. The dispense interface may only be connectable with the medical device, if the receiving opening is in the spread condition. This embodiment is inter-alia advantageous in order to prevent a connection of the dispense interface with the medical device in the relaxed condition.

Alternatively, there are also similar embodiments thinkable in which the dispense interface comprises a connector which is not arranged in the receiving opening, but in which the dispense interface may only be connectable with the medical device, if the receiving opening is in the spread condition.

According to an embodiment, the receiving opening is formed from blades arranged cylindrical and/or tapered around the connector. For instance, the blades are arranged to at least partially form a lateral surface of the receiving opening. The blades may be arranged to form the lateral surface of a truncated cone. In particular, the blades may be arranged such that their surfaces at least partially form the lateral surface of the truncated cone. For instance, the blades form a closed or an at least partially closed lateral surface of the truncated cone. The diameter of the base of this truncated cone-shaped receiving opening may be at least as big as the outer diameter of the connecting part of the medical device; and the diameter of the open, truncated end of the truncated cone-shaped receiving opening may be the opening diameter of the receiving opening.

Alternatively or additionally, the blades may form the lateral surface of a cylinder.

The connector is for instance at least partially arranged in or on the base of the receiving opening. For instance, the connector is completely encompassed in the receiving opening.

The blades may be at least partially tilting around the base of the receiving opening. For instance, the blades are made from an elastic material. The blades may be tilting such that the blades at least partially form a lateral surface of the truncated cone in a first tilt position and a lateral surface of a cylinder in a second tilt position.

By tilting the blades from the first to the second tilt position, the opening diameter of the receiving opening may be enlarged. The first tilt position may correspond to the relaxed condition of the receiving opening and the second tilt position may correspond to the spread condition of the receiving opening.

Alternatively or additionally, there are also embodiments thinkable in which the blades form a cone in the first tilt position and a truncated cone in the second tilt position or a truncated cone in the first and second tilt position.

This embodiment is inter-alia advantageous in order to form the receiving opening and/or to allow changing the condition of the receiving opening, for instance by tilting the blades.

According to an embodiment, the spreader is movable from a spreading position in the receiving opening of the dispense interface to a relaxing position in the receiving opening of the dispense interface. The spreading position may correspond to the spread condition of the receiving opening and the relaxing position may correspond to the relaxed condition of the receiving opening.

In the spreading position, the spreader is for instance arranged in the open, truncated end of the truncated cone-shaped receiving opening; and, in the relaxing position, the spreader is for instance arranged outside of the open, truncated end of the truncated cone-shaped receiving opening. In the spreading position, the maximum (cross-sectional) outer diameter of the spreader may be arranged in the plane of the open, truncated end of the truncated cone-shaped receiving opening and, in the relaxing position the maximum outer diameter of the spreader may be positioned out of the plane of the open, truncated end of the truncated cone-shaped receiving opening.

The spreader may be movable from the spreading position to the relaxing position. For instance, the spreader may be moved from the spreading position to the relaxing position by moving (e.g. pushing) the spreader entirely into the receiving opening. For instance, the spreader may be hollow such that the spreader may not block a connector arranged in the receiving opening. Alternatively or additionally, the receiving opening may comprise a recess configured to at least partially receive the spreader in the receiving opening.

Accordingly, the condition of the receiving opening may be changed from the spread condition to the relaxed condition by moving the spreader from the spreading position to the relaxing position. This embodiment is inter-alia advantageous in order to allow changing the condition of the receiving opening.

According to an embodiment, the spreader is configured to be moved from the spreading position in the receiving opening of the dispense interface to the relaxing position in the receiving opening of the dispense interface, when the connecting part of the medical device is received in the receiving opening. For instance, in the spreading position, the spreader may at least partially engage with the receiving opening and/or the receiving opening may at least partially engage with the spreader to secure the spreader in the spreading position. For instance, the spreader may comprise a notch (e.g. a circumferential notch) with which the receiving opening may engage to secure the spreader in the spreading position. In this example, the securing force is to be overcome to move the spreader from the spreading position to the relaxing position.

For instance, the connecting part of the medical device may push the spreader into the receiving opening, when the connecting part of the medical device is received in the receiving opening. Although, the spreader is moved from the spreading position to the relaxing position, the receiving opening may be in the spread condition as long as the dispense interface is attached to the medical device. For instance, the connecting part of the medical device at least partially spreads the receiving opening. After removing the dispense interface from the medical device, the spreader may remain in the relaxing position.

Accordingly, the condition of the receiving opening may be automatically changed from the spread condition to the relaxed condition, when the apparatus is removed from the medical device. A reattachment of the dispense interface to the medical device and/or a reuse of the dispense interface is prevented.

Furthermore, the position of the spreader may allow a user to determine whether the dispense interface has been used. For instance, the spreader may only viewable in the spreading position indicating that the dispense interface is unused and can be attached to a medical device. The spreader may by coloured to simplify the determination for the user.

This embodiment is inter-alia advantageous in order to automatically allow changing the condition of the receiving opening, to prevent a reuse/reattachment of the dispense interface and/or to allow a user to determine whether the dispense interface has been used.

According to an embodiment, the receiving opening is configured to lock the spreader in the relaxing position. For instance, the spreader may be secured in the relaxing position such that the spreader can not be moved from the relaxing position to the spreading position. As an example, the outer diameter of the spreader may be bigger than the opening diameter of the receiving opening in the relaxed condition. In this example, the spreader is locked in the relaxing position in the receiving opening.

This embodiment is inter-alia advantageous in order to prevent a change of the condition of the receiving opening from the relaxed condition to the spread condition.

According to an embodiment, the receiving opening is spring-loaded and/or elastic. For instance, the blades forming the lateral surface of the receiving opening may be spring-loaded. Alternatively or additionally, the blades may be made from an elastic material such as plastic.

The spring-load and/or the elasticity may force the receiving opening in the relaxed condition such that the corresponding spring-force and/or elastic-force has to be overcome to change the condition of the receiving opening from the relaxed condition to the spread condition. When the spreader and/or the connecting part of the medical device is removed from the receiving opening, the spring-load and/or elasticity may force the receiving opening back in the relaxed condition. For instance, the spring-load and/or elasticity locks the spreader in the relaxing position.

Accordingly, the condition of the receiving opening may automatically change from the spread condition to the relaxed condition, when the spreader and/or the connecting part of the medical device is removed from the receiving opening. Furthermore, a change of the condition of the receiving opening from the relaxed condition to the spread condition may be prevented.

This embodiment is inter-alia advantageous in order to automatically allow changing the condition of the receiving opening and/or to prevent a reuse/reattachment of the dispense interface.

According to an embodiment, the receiving opening has a round cross-section.

According to an embodiment, the spreader is at least partially tapered. For instance, the spreader may be a cone. The pointed base (e.g. the apex) of the spreader may allow the spreader to be easily inserted in the receiving opening; and the enlarged base may prevent the spreader from being removed from the receiving opening.

According to an embodiment, the spreader is round and/or ring-shaped. For instance, the ring-opening of a ring-shaped spreader may not block a connector arranged in the receiving opening, when the spreader is pushed in the relaxing position in the receiving opening.

According to an embodiment, an outer diameter of the spreader is at least as big as the outer diameter of the connecting part of the medical device. For instance, when the spreader is arranged in the open, truncated end of the truncated cone-shaped receiving hole, the receiving opening may be in the spread condition. When the spreader is for instance removed, the receiving opening may be in the relaxed condition.

According to an embodiment, the spreader and/or the receiving opening is made from plastic. For instance, the spreader and/or the receiving opening is a moulded plastic part. This embodiment is inter-alia advantageous in order to allow a cost effective production of the spreader and/or the receiving opening and/or to provide a biocompatible spreader and/or receiving opening.

According to an embodiment, the medical device is configured to eject a medicament.

According to an embodiment, the method further comprises moving, when receiving the connecting part of the medical device, the spreader from a spreading position in the receiving opening to a relaxing position in the receiving opening, locking the spreader in the relaxing position, and blocking the receiving the connecting part of the medical device in a relaxed condition of the receiving opening.

Accordingly, the condition of the receiving opening may automatically change from the spread condition to the relaxed condition, when the spreader and/or the connecting part of the medical device is removed from the receiving opening. Furthermore, a change of the condition of the receiving opening from the relaxed condition to the spread condition may be prevented. Furthermore, a reattachment or a reuse of the dispense interface may be prevented.

This embodiment is inter-alia advantageous in order to automatically allow changing the condition of the receiving opening and/or to prevent a reuse/reattachment of the dispense interface.

These as well as other advantages of various embodiments will become apparent to those of ordinary skill in the art by reading the following detailed description, with appropriate reference to the accompanying drawings, in which:
Fig. 1 illustrates a perspective view of the delivery device illustrated in Fig. 1a and 1b with an end cap of the device removed;
Fig. 2 illustrates a perspective view of the delivery device distal end showing the cartridge;
Fig. 3 illustrates a perspective view of the cartridge holder illustrated in Fig. 1 with one cartridge retainer in an open position;
Fig. 4 illustrates a dispense interface and a dose dispenser that may be removably attached to a distal end of the delivery device illustrated in Fig. 1;
Fig. 5 illustrates the dispense interface and the dose dispenser illustrated in Fig. 4 attached to a distal end of the delivery device illustrated in Fig. 1;
Fig. 6 illustrates one arrangement of the dose dispenser without a reuse protection that may be attached to a distal end of the delivery device;
Fig. 7 illustrates a perspective view of the dispense interface illustrated in Fig. 4;
Fig. 8 illustrates another perspective view of the dispense interface without a reuse protection illustrated in Fig. 4;
Fig. 9 illustrates a cross-sectional view of the dispense interface without a reuse protection illustrated in Fig. 4;
Fig. 10 illustrates an exploded view of the dispense without a reuse protection interface illustrated in Fig. 4;
Fig. 11 illustrates a cross-sectional view of the dispense interface without a reuse protection and dose dispenser without a reuse protection attached to a drug delivery device, such as the device illustrated in Fig. 1;
Fig. 12 illustrates an embodiment of a receiving opening of a reuse protection for the dispense interface illustrated in Fig. 4;
Fig. 13 illustrates cross-sectional views of the receiving opening illustrated in Fig. 12 mounted on the dispense interface illustrated in Fig. 4;

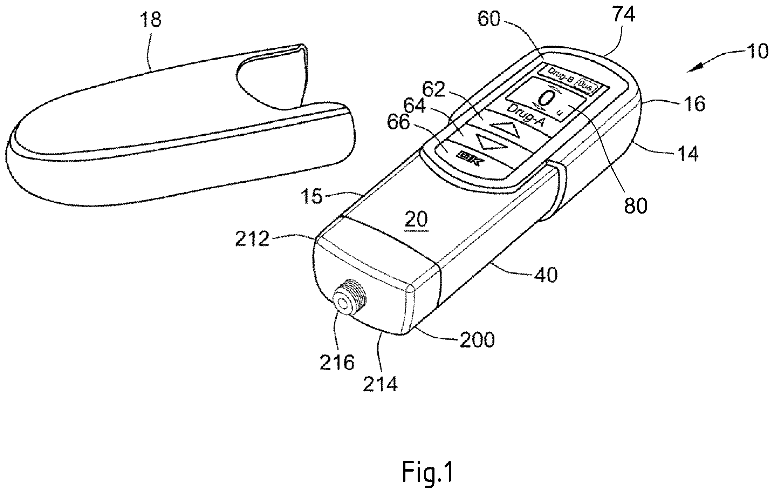

The drug delivery device illustrated in Fig. 1 comprises a main body 14 that extends from a proximal end 16 to a distal end 15. At the distal end 15, a removable end cap or cover 18 is provided. This end cap 18 and the distal end 15 of the main body 14 work together to provide a snap fit or form fit connection so that once the cover 18 is slid onto the distal end 15 of the main body 14, this frictional fit between the cap and the main body outer surface 20 prevents the cover from inadvertently falling off the main body.

The main body 14 contains a micro-processor control unit, an electro-mechanical drive train, and at least two medicament reservoirs. When the end cap or cover 18 is removed from the device 10 (as illustrated in Fig. 1), a dispense interface 200 is attached to the distal end 15 of the main body 14, and a dose dispenser (e.g., a needle assembly) is attached to the interface. The drug delivery device 10 can be used to administer a computed dose of a second medicament (secondary drug compound) and a variable dose of a first medicament (primary drug compound) through a single needle assembly, such as a double ended needle assembly.

A control panel region 60 is provided near the proximal end of the main body 14. Preferably, this control panel region 60 comprises a digital display 80 along with a plurality of human interface elements that can be manipulated by a user to set and inject a combined dose. In this arrangement, the control panel region comprises a first dose setting button 62, a second dose setting button 64 and a third button 66 designated with the symbol "OK." In addition, along the most proximal end of the main body, an injection button 74 is also provided (not visible in the perspective view of Fig. 1).

The cartridge holder 40 can be removably attached to the main body 14 and may contain at least two cartridge retainers 50 and 52. Each retainer is configured so as to contain one medicament reservoir, such as a glass cartridge. Preferably, each cartridge contains a different medicament.

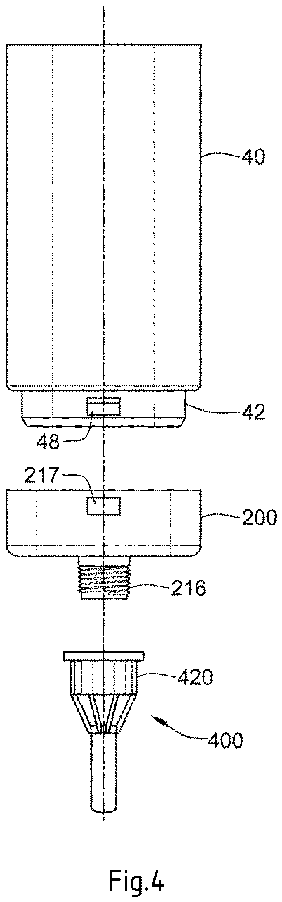

In addition, at the distal end of the cartridge holder 40, the drug delivery device illustrated in Fig. 1 includes a dispense interface 200. As will be described in relation to Fig. 4, in one arrangement, this dispense interface 200 includes a main outer body 212 that is removably attached to a distal end 42 of the cartridge housing 40. As can be seen in Fig. 1, a distal end 214 of the dispense interface 200 preferably comprises a needle hub 216. This needle hub 216 may be configured so as to allow a dose dispenser, such as a conventional pen type injection needle assembly, to be removably attached to the drug delivery device 10.

Once the device is turned on, the digital display 80 shown in Fig. 1 illuminates and provides the user certain device information, preferably information relating to the medicaments contained within the cartridge holder 40. For example, the user is provided with certain information relating to both the primary medicament (Drug A) and the secondary medicament (Drug B).

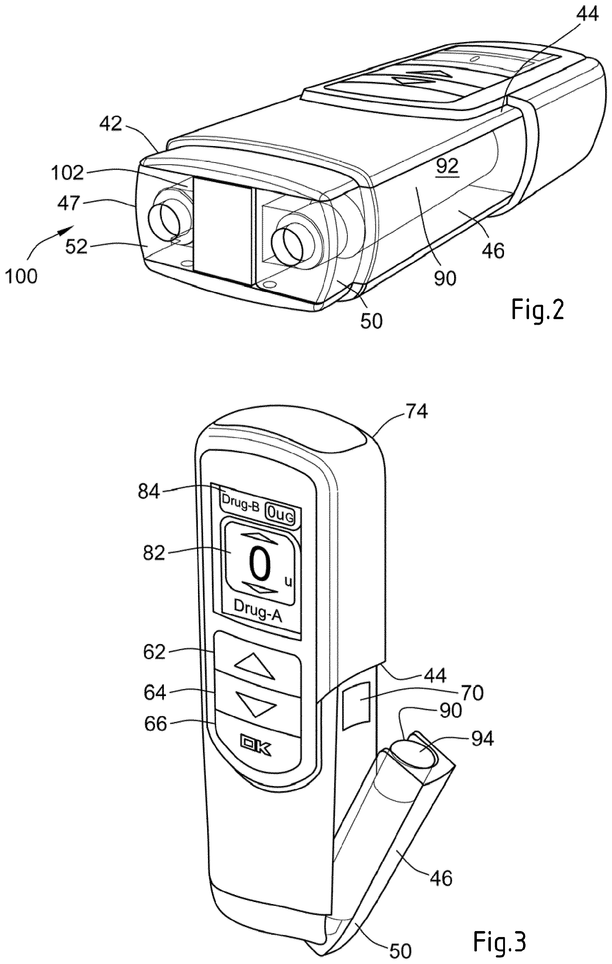

As shown in Fig. 3, the first and a second cartridge retainers 50, 52 comprise hinged cartridge retainers. These hinged retainers allow user access to the cartridges. Fig. 3 illustrates a perspective view of the cartridge holder 40 illustrated in Fig. 1 with the first hinged cartridge retainer 50 in an open position. Fig. 3 illustrates how a user might access the first cartridge 90 by opening up the first retainer 50 and thereby having access to the first cartridge 90.

As mentioned above when discussing Fig. 1, a dispense interface 200 is coupled to the distal end of the cartridge holder 40. Fig. 4 illustrates a flat view of the dispense interface 200 unconnected to the distal end of the cartridge holder 40. A dose dispenser or needle assembly that may be used with the interface 200 is also illustrated and is provided in a protective outer cap 420.

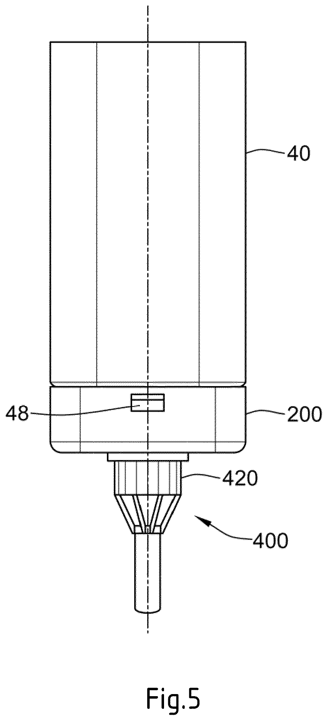

In Fig. 5, the dispense interface 200 illustrated in Fig. 4 is shown coupled to the cartridge holder 40. The axial attachment means between the dispense interface 200 and the cartridge holder 40 can be any known axial attachment means to those skilled in the art, including snap locks, snap fits, snap rings, keyed slots, and combinations of such connections. The connection or attachment between the dispense interface and the cartridge holder may also contain additional features (not shown), such as connectors, stops, splines, ribs, grooves, pips, clips and the like design features, that ensure that specific hubs are attachable only to matching drug delivery devices. Such additional features would prevent the insertion of a non-appropriate secondary cartridge to a non-matching injection device.

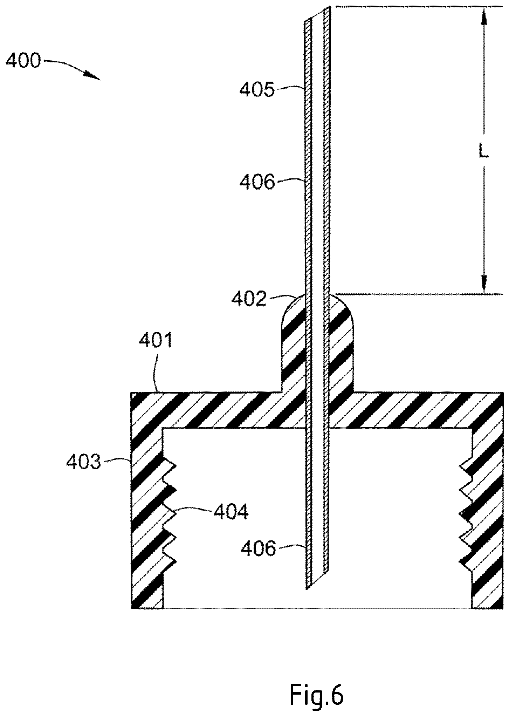

Fig. 5 also illustrates the needle assembly 400 and protective cover 420 coupled to the distal end of the dispense interface 200 that may be screwed onto the needle hub of the interface 200. Fig. 6 illustrates a cross sectional view of the double ended needle assembly 402 attached to the dispense interface 200 in Fig. 5.

The needle assembly 400 illustrated in Fig. 6 comprises a double ended needle 406 and a hub 401. The double ended needle or cannula 406 is fixedly mounted in a needle hub 401. This needle hub 401 comprises a circular disk shaped element which has along its periphery a circumferential depending sleeve 403. Along an inner wall of this hub member 401, a thread 404 is provided. This thread 404 allows the needle hub 401 to be screwed onto the dispense interface 200 which, in one embodiment, is provided with a corresponding outer thread along a distal hub. At a center portion of the hub element 401 there is provided a protrusion 402. This protrusion 402 projects from the hub in an opposite direction of the sleeve member. A double ended needle 406 is mounted centrally through the protrusion 402 and the needle hub 401. This double ended needle 406 is mounted such that a first or distal piercing end 405 of the double ended needle forms an injecting part for piercing an injection site (e.g., the skin of a user).

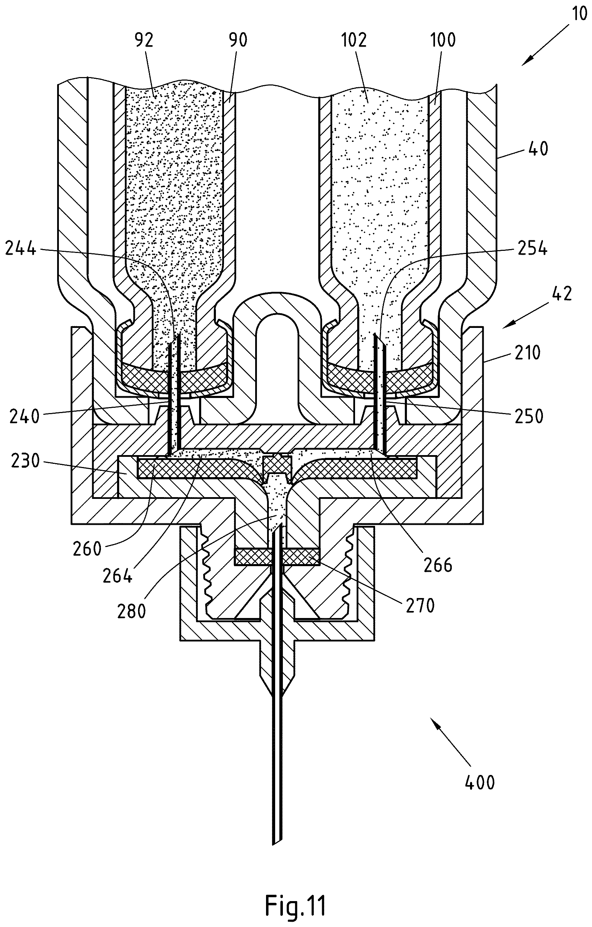

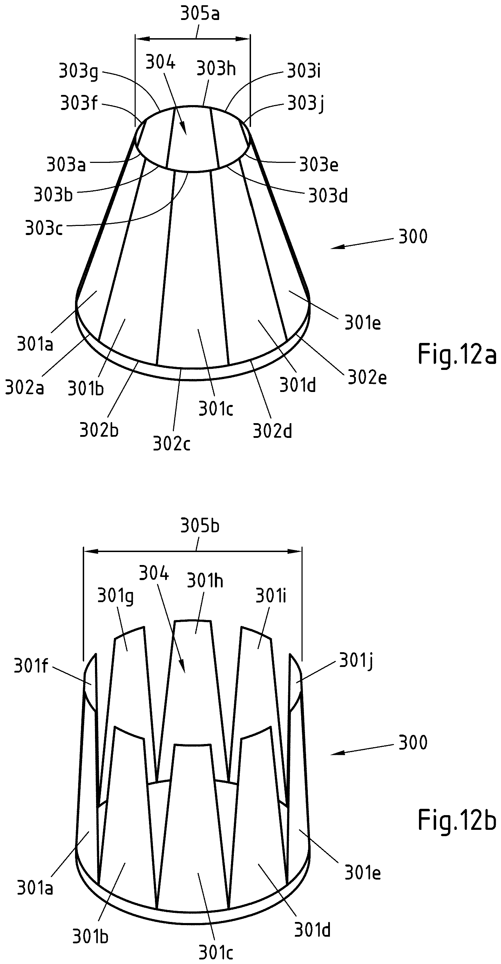

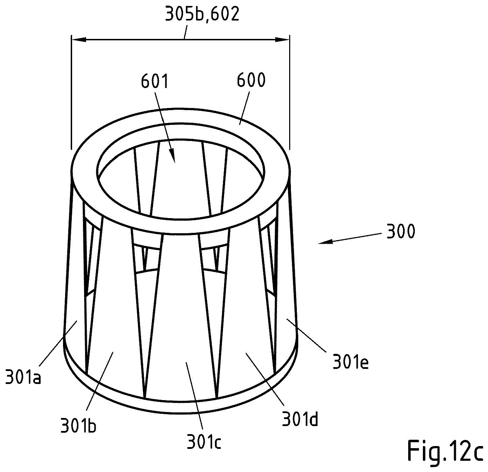

Similarly, a second or proximal piercing end 406 of the needle assembly 400 protrudes from an opposite side of the circular disc so that it is concentrically surrounded by the sleeve 403. In one needle assembly arrangement, the second or proximal piercing end 406 may be shorter than the sleeve 403 so that this sleeve to some extent protects the pointed end of the back sleeve. The needle cover cap 420 illustrated in Fig. 11 and 12 provides a form fit around the outer surface 403 of the hub 401.
Referring now to Fig. 4-11, one embodiment of this interface 200 will now be discussed. In this one embodiment, this interface 200 comprises:
a. a main outer body 210,
b. an first inner body 220,
c. a second inner body 230,
d. a first piercing needle 240,
e. a second piercing needle 250,
f. a valve seal 260, and
g. a septum 270.

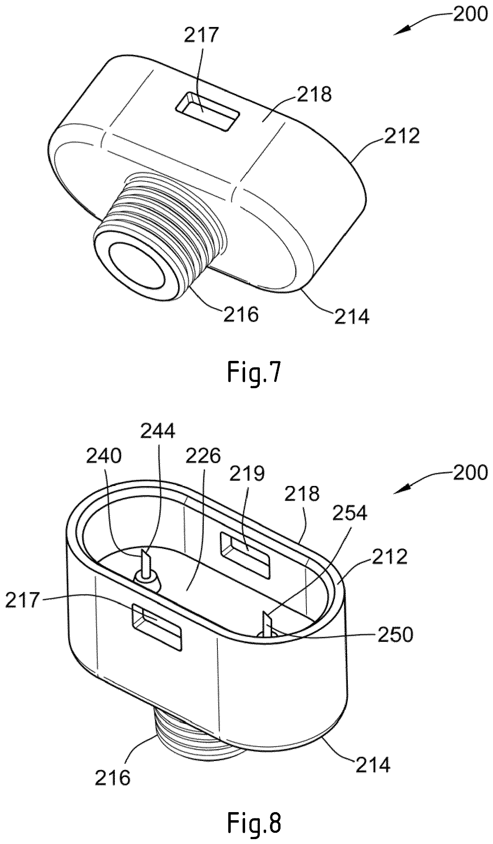

The main outer body 210 comprises a main body proximal end 212 and a main body distal end 214. At the proximal end 212 of the outer body 210, a connecting member is configured so as to allow the dispense interface 200 to be attached to the distal end of the cartridge holder 40. Preferably, the connecting member is configured so as to allow the dispense interface 200 to be removably connected the cartridge holder 40. In one preferred interface arrangement, the proximal end of the interface 200 is configured with an upwardly extending wall 218 having at least one recess. For example, as may be seen from Fig. 8, the upwardly extending wall 218 comprises at least a first recess 217 and a second recess 219.

Preferably, the first and the second recesses 217, 219 are positioned within this main outer body wall so as to cooperate with an outwardly protruding member located near the distal end of the cartridge housing 40 of the drug delivery device 10. For example, this outwardly protruding member 48 of the cartridge housing may be seen in Fig. 4 and 5. A second similar protruding member is provided on the opposite side of the cartridge housing. As such, when the interface 200 is axially slid over the distal end of the cartridge housing 40, the outwardly protruding members will cooperate with the first and second recess 217, 219 to form an interference fit, form fit, or snap lock. Alternatively, and as those of skill in the art will recognize, any other similar connection mechanism that allows for the dispense interface and the cartridge housing 40 to be axially coupled could be used as well.

The main outer body 210 and the distal end of the cartridge holder 40 act to form an axially engaging snap lock or snap fit arrangement that could be axially slid onto the distal end of the cartridge housing. In one alternative arrangement, the dispense interface 200 may be provided with a coding feature so as to prevent inadvertent dispense interface cross use. That is, the inner body of the hub could be geometrically configured so as to prevent an inadvertent cross use of one or more dispense interfaces.

A mounting hub is provided at a distal end of the main outer body 210 of the dispense interface 200. Such a mounting hub can be configured to be releasably connected to a needle assembly. As just one example, this connecting means 216 may comprise an outer thread that engages an inner thread provided along an inner wall surface of a needle hub of a needle assembly, such as the needle assembly 400 illustrated in Fig. 6. Alternative releasable connectors may also be provided such as a snap lock, a snap lock released through threads, a bayonet lock, a form fit, or other similar connection arrangements.

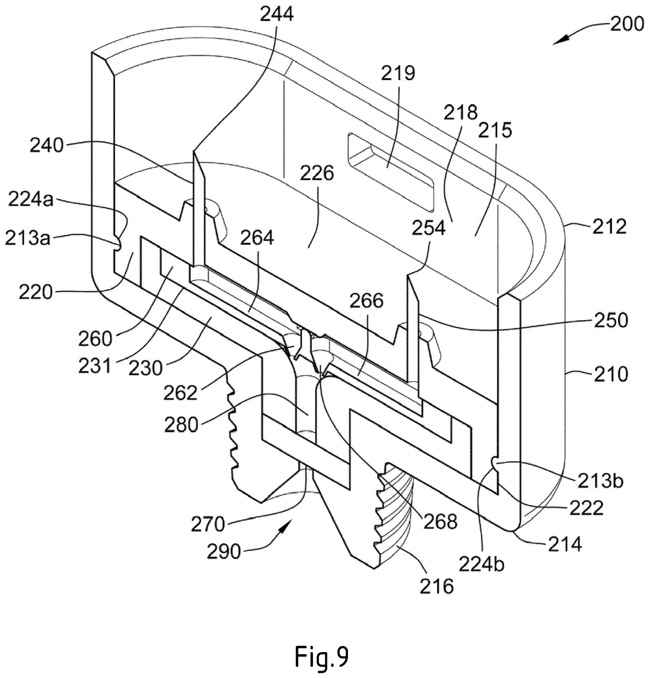

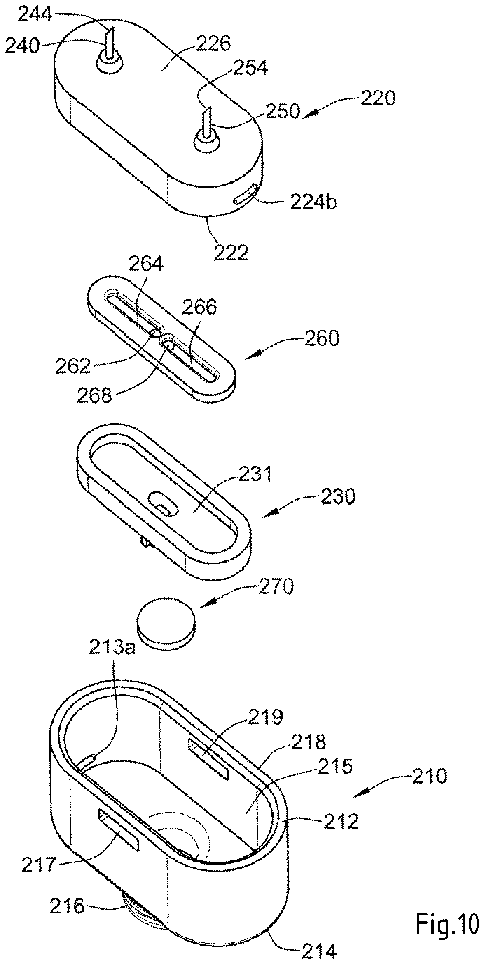

The dispense interface 200 further comprises a first inner body 220. Certain details of this inner body are illustrated in Fig. 8-11. Preferably, this first inner body 220 is coupled to an inner surface 215 of the extending wall 218 of the main outer body 210. More preferably, this first inner body 220 is coupled by way of a rib and groove form fit arrangement to an inner surface of the outer body 210. For example, as can be seen from Fig. 9, the extending wall 218 of the main outer body 210 is provided with a first rib 213a and a second rib 213b. This first rib 213a is also illustrated in Fig. 10. These ribs 213a and 213b are positioned along the inner surface 215 of the wall 218 of the outer body 210 and create a form fit or snap lock engagement with cooperating grooves 224a and 224b of the first inner body 220. In an embodiment, these cooperating grooves 224a and 224b are provided along an outer surface 222 of the first inner body 220.

In addition, as can be seen in Fig. 8-10, a proximal surface 226 near the proximal end of the first inner body 220 may be configured with at least a first proximally positioned piercing needle 240 comprising a proximal piercing end portion 244. Similarly, the first inner body 220 is configured with a second proximally positioned piercing needle 250 comprising a proximally piercing end portion 254. Both the first and second needles 240, 250 are rigidly mounted on the proximal surface 226 of the first inner body 220.

Preferably, this dispense interface 200 further comprises a valve arrangement. Such a valve arrangement could be constructed so as to prevent cross contamination of the first and second medicaments contained in the first and second reservoirs, respectively. A preferred valve arrangement may also be configured so as to prevent back flow and cross contamination of the first and second medicaments.

In one preferred system, dispense interface 200 includes a valve arrangement in the form of a valve seal 260. Such a valve seal 260 may be provided within a cavity 231 defined by the second inner body 230, so as to form a holding chamber 280. Preferably, cavity 231 resides along an upper surface of the second inner body 230. This valve seal comprises an upper surface that defines both a first fluid groove 264 and second fluid groove 266. For example, Fig. 9 illustrates the position of the valve seal 260, seated between the first inner body 220 and the second inner body 230. During an injection step, this seal valve 260 helps to prevent the primary medicament in the first pathway from migrating to the secondary medicament in the second pathway, while also preventing the secondary medicament in the second pathway from migrating to the primary medicament in the first pathway. Preferably, this seal valve 260 comprises a first non-return valve 262 and a second non-return valve 268. As such, the first non-return valve 262 prevents fluid transferring along the first fluid pathway 264, for example a groove in the seal valve 260, from returning back into this pathway 264. Similarly, the second non-return valve 268 prevents fluid transferring along the second fluid pathway 266 from returning back into this pathway 266.

Together, the first and second grooves 264, 266 converge towards the non-return valves 262 and 268 respectively, to then provide for an output fluid path or a holding chamber 280. This holding chamber 280 is defined by an inner chamber defined by a distal end of the second inner body both the first and the second non return valves 262, 268 along with a pierceable septum 270. As illustrated, this pierceable septum 270 is positioned between a distal end portion of the second inner body 230 and an inner surface defined by the needle hub of the main outer body 210.

The holding chamber 280 terminates at an outlet port of the interface 200. This outlet port 290 is preferably centrally located in the needle hub of the interface 200 and assists in maintaining the pierceable seal 270 in a stationary position. As such, when a double ended needle assembly is attached to the needle hub of the interface (such as the double ended needle illustrated in Fig. 6), the output fluid path allows both medicaments to be in fluid communication with the attached needle assembly.

The hub interface 200 further comprises a second inner body 230. As can be seen from Fig. 9, this second inner body 230 has an upper surface that defines a recess, and the valve seal 260 is positioned within this recess. Therefore, when the interface 200 is assembled as shown in Fig. 9, the second inner body 230 will be positioned between a distal end of the outer body 210 and the first inner body 220. Together, second inner body 230 and the main outer body hold the septum 270 in place. The distal end of the inner body 230 may also form a cavity or holding chamber that can be configured to be fluid communication with both the first groove 264 and the second groove 266 of the valve seal.

Axially sliding the main outer body 210 over the distal end of the drug delivery device attaches the dispense interface 200 to the multi-use device. In this manner, a fluid communication may be created between the first needle 240 and the second needle 250 with the primary medicament of the first cartridge and the secondary medicament of the second cartridge, respectively.

Fig. 11 illustrates the dispense interface 200 after it has been attached to the distal end 42 of the cartridge holder 40 of the drug delivery device 10 illustrated in Fig. 1. A double ended needle 400 is also attached to the distal end of this interface. The cartridge holder 40 is illustrated as having a first cartridge containing a first medicament and a second cartridge containing a second medicament.

When the interface 200 is first attached to the distal end of the cartridge holder 40, the proximal piercing end 244 of the first piercing needle 240 pierces the septum of the first cartridge 90 and thereby resides in fluid communication with the primary medicament 92 of the first cartridge 90. A distal end of the first piercing needle 240 will also be in fluid communication with a first fluid path groove 264 defined by the valve seal 260.

Similarly, the proximal piercing end 254 of the second piercing needle 250 pierces the septum of the second cartridge 100 and thereby resides in fluid communication with the secondary medicament 102 of the second cartridge 100. A distal end of this second piercing needle 250 will also be in fluid communication with a second fluid path groove 266 defined by the valve seal 260.

Fig. 11 illustrates an embodiment of such a dispense interface 200 that is coupled to a distal end 15 of the main body 14 of drug delivery device 10. Preferably, such a dispense interface 200 is removably coupled to the cartridge holder 40 of the drug delivery device 10.

As illustrated in Fig. 11, the dispense interface 200 is coupled to the distal end of a cartridge housing 40. This cartridge holder 40 is illustrated as containing the first cartridge 90 containing the primary medicament 92 and the second cartridge 100 containing the secondary medicament 102. Once coupled to the cartridge housing 40, the dispense interface 200 essentially provides a mechanism for providing a fluid communication path from the first and second cartridges 90, 100 to the common holding chamber 280. This holding chamber 280 is illustrated as being in fluid communication with a dose dispenser. Here, as illustrated, this dose dispenser comprises the double ended needle assembly 400. As illustrated, the proximal end of the double ended needle assembly is in fluid communication with the chamber 280.

In one embodiment, the dispense interface is configured so that it attaches to the main body in only one orientation, that is it is fitted only one way round. As such as illustrated in Fig. 11, once the dispense interface 200 is attached to the cartridge holder 40, the primary needle 240 can only be used for fluid communication with the primary medicament 92 of the first cartridge 90 and the interface 200 would be prevented from being reattached to the holder 40 so that the primary needle 240 could now be used for fluid communication with the secondary medicament 102 of the second cartridge 100. Such a one way around connecting mechanism may help to reduce potential cross contamination between the two medicaments 92 and 102.

Fig. 12a and b illustrate an embodiment of a receiving opening 300 of a reuse protection for the dispense interface illustrated in Fig. 4. For instance, the receiving opening 300 comprising the blades 302a-302j is a moulded plastic part.

As illustrated in Fig. 12a and b, the lateral surface of the receiving opening 300 is formed from blades 301 (i.e. blades 301a-301j). In Fig. 12a and b the blades 301 form the lateral surface of a truncated cone. Accordingly, the receiving opening 300 is truncated cone-shaped. Therein, the truncated end of the receiving opening 300 is open (see opening 304). The opening 304 has the opening diameter 305 (e.g. opening diameters 305a and b). The blades 301 have distal edges 302 (i.e. edges 302a-302j, respectively). The distal edges 302 of the blades 301 are tilting edges such that the blades 301 are elastically tilting around the distal edges 302. The elasticity causes an elastic force that constricts the blades 301, for instance in the direction of the center of opening 304. The blades 301 have proximal edges 303 (i.e. edges 303a-303j, respectively), which define the opening 304.

In Fig. 12a, the blades 301 are in a first tilt position. Therein, the adjacent edges of the blades 301 are meeting such that the lateral surface of the receiving opening 300 is closed and the blades 301 are constricted. Thus, the receiving opening 300 is in a relaxed condition and the opening 304 has the minimum opening diameter 305a.

In Fig. 12b, the blades 301 are in a second tilt position and form the lateral surface of the receiving opening 300. Therein, the lateral surface of the receiving opening 300 is partially opened and opening 304 has the opening diameter 305b. The opening diameter 305b is bigger than the opening diameter 305a. Thus, the receiving opening is in a spread condition.

Due to the elastic force constricting the blades 301, a counterforce pushing apart the blades 301 has to be applied to maintain the opening diameter 305b of the receiving opening 300; otherwise, the blades 301 will return to the first tilt position.

In Fig. 12c, a ring-shaped spreader 600 is inserted in opening 304 of the receiving opening 300. Therein, the proximal edges 303 of the blades 301 are forced in touch with the lateral surface of the spreader 600 by the elastic force as described above. The spreader has a ring-opening 601. Since the outer diameter 602 of the spreader 600 equals the opening diameter 305b of the receiving opening 300 illustrated in Fig. 12b, the spreader 600 pushes apart (i.e. spreads) the blades 301 and prevent the blades 301 from returning to the first tilt position. The opening diameter 305b of opening 304 is maintained as long as the spreader is inserted in the opening 304 of the receiving opening. Accordingly, this position of the spreader 600 is called spreading position.

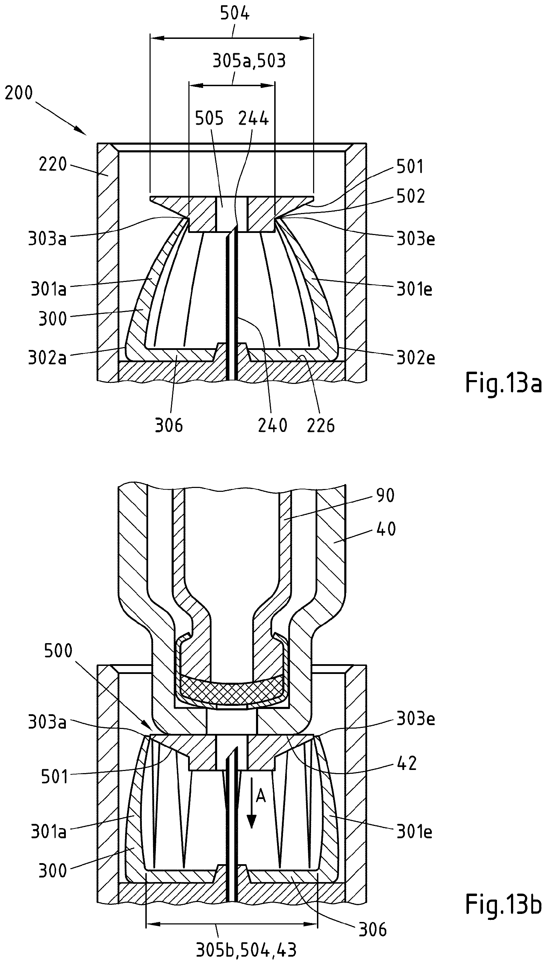

Fig. 13a to d illustrate a cross-sectional view of the receiving opening 300 illustrated in Fig. 12a and b mounted on the dispense interface 200 illustrated in Fig. 4.

In Fig. 13a to d, the receiving opening 300 is mounted on the proximal surface 226 of the dispense interface 200. Therein, the receiving opening 300 at least partially encompasses the first proximally positioned piercing needle 240 and the proximal piercing end portion 244 thereof. Another, receiving opening 300 may similarly encompass the second proximally positioned piercing needle 250.

A spreader 500 is inserted in opening 304 of the receiving opening 300. The lateral surface of the spreader 500 is at least partially formed as a truncated cone with a tapered surface 501 and a notch 502. Alternatively, the lateral surface of the spreader 500 may at least partially be formed as a truncated cone with a tapered surface 501 but without a notch 502. Alternatively or additionally, the lateral surface of the spreader 500 may at least partially be formed as a cylinder (e.g. the spreader may be the ring-shaped spreader illustrated in Fig. 12c).

The notch 502 is arranged at the truncated end of spreader 500. At the truncated end and/or at the notch 502, the spreader 500 has the minimum outer diameter 503. The minimum outer diameter 503 of the spreader 500 equals the minimum opening diameter 305a of the receiving opening 300 illustrated in Fig. 12a.

At the base, the spreader 500 has the maximum outer diameter 504. The maximum outer diameter 504 equals the opening diameter 305b of the receiving opening 300 illustrated in Fig. 12b. Furthermore, spreader 500 is hollow and has a longitudinal opening 505.

As illustrated in Fig. 13a, the proximal edges 303 of the blades 301 engage with the notch 502 such that the receiving opening is in the relaxed condition illustrated in Fig. 12. Therein, the spreader 500 is secured in this position by the proximal edges 303 of the blades 301 forced in engagement with notch 502 by the elastic force as described above. For instance, the spreader 500 may be arranged in the receiving opening 300 as illustrated in Fig. 13a during the production of the dispense interface 200.

Fig. 13b illustrates the situation, during the attachment of the dispense interface 200 illustrated in Fig. 13a to the drug delivery device 10 illustrated in Fig. 1. In particular, the dispense interface 200 is attached to the distal end 42 of the cartridge holder 40 of the drug delivery device 10 as described above.

In order to attach the dispense interface 200 to the drug delivery device 10, the distal end 42 of the cartridge holder 40 relatively moves in the direction A. Therein, the distal end 42 of the cartridge holder 40 pushes the spreader 500 in the direction A into the receiving opening. Due to the movement of the spreader 500 in the direction A, the proximal edges 303 of blades 301 slip along the tapered surface 501 of the spreader 500 such that the receiving opening 300 is spread by the spreader 500 and the opening diameter 305 of the receiving opening 300 enlarges.

The use of a spreader with a tapered surface and a notch is inter-alia advantageous in order to prevent a deterioration of the elasticity of the receiving opening, which may appear if the receiving opening is spread for a longer time such as the time between the production of the dispense interface and the attachment of the dispense interface to a drug delivery device.

As illustrated in Fig. 13b, the proximal edges 303 encompass the base of the spreader 500. The diameter of the base of the spreader is the maximum outer diameter 504 of the spreader 500 and equals the opening diameter 305b of the receiving opening 300 illustrated in Fig. 12b. Furthermore, the maximum outer diameter 504 of the spreader 500 equals the outer diameter 43 of the distal end 42 of the cartridge holder 40. Thus, the receiving opening 300 is in the spread condition, the spreader 500 is in the spreading position and the distal end 42 of the cartridge holder 40 is receivable in the receiving opening 300. The diameter of the base 306 of the receiving opening 300 is at least as big as the opening diameter 305b of the receiving opening 300.

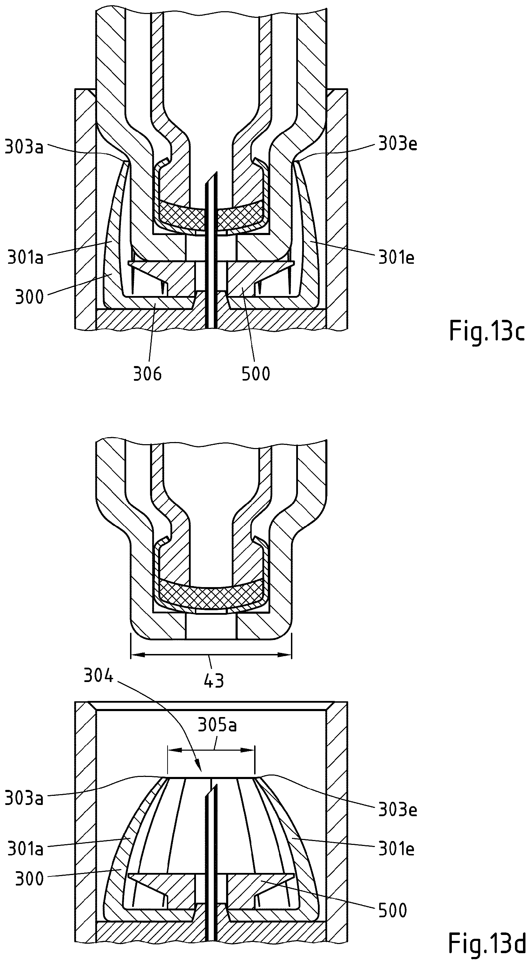

Fig. 13c illustrates the situation, when the dispense interface 200 illustrated in Fig. 13a is attached to the drug delivery device 10 illustrated in Fig. 1. As illustrated in Fig. 13c, the distal end 42 of the cartridge holder 40 is received in the receiving opening 300. Therein, the proximal edges 303 of the blades 301 touch the distal end 42 of the cartridge holder 40 and the spreader is arranged at the base 306 of the receiving opening 300. The first proximally positioned piercing needle 240 of the dispense interface protrudes through opening 505 of the spreader 500. The proximal piercing end portion 244 of the first proximally positioned piercing needle 240 pierces the septum of the first cartridge 90 and thereby resides in fluid communication with the primary medicament 92 of the first cartridge 90 as described above.

Fig. 13d illustrates the situation, when the dispense interface 200 illustrated in Fig. 13a is removed from the drug delivery device 10 illustrated in Fig. 1. Therein, the receiving opening 300 is returned to the relaxed condition illustrated in Fig. 12a by the elastic force as described above. The blades 301 are constricted and lock the spreader 500 at the base 306 of the receiving opening. The opening diameter 305a is smaller than the outer diameter 43 of the distal end 42 of the cartridge holder 40. Thus, the spreader 500 is locked in the relaxing position and a reattachment of the dispense interface 200 to the drug delivery device 10 is prevented.

As illustrated in Fig. 13a to d, the reuse protection comprising the receiving opening 300 prevents the reuse (e.g. the reattachment) of the dispense interface 200. The reuse protection illustrated in Fig. 13a to d is not limited to the dispense interface 200 as illustrated in Fig. 4. The reuse protection may also be used to prevent the reuse of a needle assembly such as needle assembly 400.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(O2)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(O2)25, Asp28] Exendin-4(1-39)-NH2,
   des Pro36, Pro37, Pro38 [Met(O)14, Trp(O2)25, Asp28] Exendin-4(1-39)-(Lys)6-NH2,
   H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
   or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

## Claims

1. A dispense interface (200) attachable to a medical device (10), wherein the dispense interface (200) comprises:
a receiving opening (300) comprising blades (301) arranged to form an opening (304) and to be constricted in the direction of the centre of the opening (304) by an elastic force,
the receiving opening (300) having a closed condition and a spread condition,
wherein in the closed condition the opening (304) has an opening diameter (305) that is smaller than an outer diameter (43) of a connecting part (42) of the medical device (10), and
in the spread condition the opening diameter (305) of the opening (304) is at least as big as the outer diameter (43) of the connecting part (42) of the medical device (10), and
the receiving opening (300) is configured to receive the connecting part (42) of the medical device (10) when in the spread condition and to block receiving said connecting part (42) of said medical device (10) when in the closed condition; and
a spreader (500,600) that is at least partially tapered and insertable into the receiving opening (300) and
the spreader (500,600) is configured, during insertion, to move into the receiving opening (300) such that the blades (301) slip along the at least partially tapered surface of the spreader (500,600) to spread the blades (301) of said receiving opening (300) and enlarge an opening diameter (305) of the receiving opening (300), when the receiving opening (300) is in the spread condition.

2. The dispense interface according to claim 1, wherein said spreader (500,600) is further configured to allow the blades (301) of said receiving opening (300) to close in a direction of a centre of the opening (304) when said receiving opening (300) is in the closed condition.

3. The dispense interface according to any of the claims 1 to 2, said apparatus further comprising:
a connector (244) configured to connect to said connecting part (42) of said medical device (10), wherein said connector (244) is arranged in said receiving opening (300).

4. The dispense interface according to claim 3, wherein the blades (301) are arranged cylindrical and/or tapered around said connector (244).

5. The dispense interface according to any of the claims 1 to 4, wherein said spreader (500,600) is configured to move from a first position in said receiving opening (300) of said apparatus to a second position in said receiving opening (300) of said apparatus.

6. The dispense interface according to claim 5, wherein when the spreader (500,600) is in the second position, said receiving opening (300) is configured to lock said spreader (500,600) at the base of the receiving opening (300).

7. The dispense interface according to any of the claims 1 to 6, wherein said receiving opening (300) is spring-loaded.

8. The dispense interface according to any of the claims 1 to 7, wherein said receiving opening (300) has a round cross-section.

9. The dispense interface according to any of the claims 1 to 8, wherein said spreader (500,600) is round and/or ring-shaped.

10. The dispense interface according to any of the claims 1 to 9, wherein an outer diameter (504) of said spreader (500,600) is at least as big as said outer diameter (43) of said connecting part (42) of said medical device (10).

11. The dispense interface according to any of the claims 1 to 10, wherein said receiving opening (300) and/or said spreader (500,600) is made from plastic.

12. The dispense interface according to any of the claims 1 to 11, wherein said medical device (10) is configured to eject a medicament.

13. A method of reuse protection for a dispense interface (200) according to any of the preceding claims, the method comprising:
spreading, by the spreader (500,600), the blades (301) of the receiving opening (300) when the receiving opening (300) is in the spread condition, such that the opening diameter (305) of the opening (304) is at least as big as the outer diameter (43) of the connecting part (42) of the medical device (10);
receiving, when the receiving opening (300) is in the spread condition, in said receiving opening (300), the connecting part (42) of the medical device (10);
moving, when receiving said connecting part (42) of said medical device (10), said spreader (500,600) from a first position in said receiving opening (300) to a second position in said receiving opening (300);
locking, when the spreader (500,600) is in the second position, said spreader (500,600) at the base of the receiving opening (300); and
blocking, when the receiving opening (300) is in the closed condition, said receiving said connecting part (42) of said medical device (10).

## Patentansprüche

1. Abgabeschnittstelle (200), die an einer medizinischen Vorrichtung (10) befestigbar ist,
wobei die Abgabeschnittstelle (200) das Folgende umfasst:
eine Aufnahmeöffnung (300), die Klingen (301) umfasst, die so angeordnet sind, dass sie eine Öffnung (304) bilden und in Richtung der Mitte der Öffnung (304) durch eine elastische Kraft zusammenziehbar sind,
wobei die Aufnahmeöffnung (300) einen geschlossenen Zustand und einen gespreizten Zustand aufweist,
wobei die Öffnung (304) im geschlossenen Zustand einen Öffnungsdurchmesser (305) aufweist, der kleiner als ein Außendurchmesser (43) eines Verbindungsteils (42) der medizinischen Vorrichtung (10) ist, und
wobei der Öffnungsdurchmesser (305) der Öffnung (304) im gespreizten Zustand zumindest so groß wie der Außendurchmesser (43) des Verbindungsteils (42) der medizinischen Vorrichtung (10) ist, und
wobei die Aufnahmeöffnung (300) dazu ausgelegt ist, das Verbindungsteil (42) der medizinischen Vorrichtung (10) im gespreizten Zustand aufzunehmen und die Aufnahme des Verbindungsteils (42) der medizinischen Vorrichtung (10) im geschlossenen Zustand zu blockieren; und
einen Spreizer (500, 600), der zumindest teilweise verjüngt ist und in die Aufnahmeöffnung (300) einführbar ist, und
wobei der Spreizer (500, 600) während der Einführung dazu ausgelegt ist, sich so in die Aufnahmeöffnung (300) zu bewegen, dass die Klingen (301) entlang der zumindest teilweise verjüngten Oberfläche des Spreizers (500, 600) gleiten, um die Klingen (301) der Aufnahmeöffnung (300) zu spreizen und einen Öffnungsdurchmesser (305) der Aufnahmeöffnung (300) zu vergrößern, wenn sich die Aufnahmeöffnung (300) im gespreizten Zustand befindet.

2. Abgabeschnittstelle nach Anspruch 1, wobei der Spreizer (500, 600) ferner dazu ausgelegt ist, es den Klingen (301) der Aufnahmeöffnung (300) zu erlauben, sich in einer Richtung einer Mitte der Öffnung (304) zu schließen, wenn sich die Aufnahmeöffnung (300) im geschlossenen Zustand befindet.

3. Abgabeschnittstelle nach einem der Ansprüche 1 bis 2, wobei die Vorrichtung ferner das Folgende umfasst:
ein Verbindungsglied (244), das zur Verbindung des Verbindungsteils (42) der medizinischen Vorrichtung (10) ausgelegt ist, wobei das Verbindungsglied (244) in der Aufnahmeöffnung (300) angeordnet ist.

4. Abgabeschnittstelle nach Anspruch 3, wobei die Klingen (301) zylindrisch und/oder verjüngt um das Verbindungsglied (244) angeordnet sind.

5. Abgabeschnittstelle nach einem der Ansprüche 1 bis 4, wobei der Spreizer (500, 600) dazu ausgelegt ist, sich aus einer ersten Position in der Aufnahmeöffnung (300) der Vorrichtung in eine zweite Position in der Aufnahmeöffnung (300) der Vorrichtung zu bewegen.

6. Abgabeschnittstelle nach Anspruch 5, wobei die Aufnahmeöffnung (300), wenn sich der Spreizer (500, 600) in der zweiten Position befindet, dazu ausgelegt ist, den Spreizer (500, 600) an der Basis der Aufnahmeöffnung (300) zu arretieren.

7. Abgabeschnittstelle nach einem der Ansprüche 1 bis 6, wobei die Aufnahmeöffnung (300) federbelastet ist.

8. Abgabeschnittstelle nach einem der Ansprüche 1 bis 7, wobei die Aufnahmeöffnung (300) einen runden Querschnitt aufweist.

9. Abgabeschnittstelle nach einem der Ansprüche 1 bis 8, wobei der Spreizer (500, 600) rund und/oder ringförmig ist.

10. Abgabeschnittstelle nach einem der Ansprüche 1 bis 9, wobei ein Außendurchmesser (504) des Spreizers (500, 600) zumindest so groß wie der Außendurchmesser (43) des Verbindungsteils (42) der medizinischen Vorrichtung (10) ist.

11. Abgabeschnittstelle nach einem der Ansprüche 1 bis 10, wobei die Aufnahmeöffnung (300) und/oder der Spreizer (500, 600) aus Kunststoff gefertigt sind.

12. Abgabeschnittstelle nach einem der Ansprüche 1 bis 11, wobei die medizinische Vorrichtung (10) zum Ausstoßen eines Medikaments ausgelegt ist.

13. Verfahren zum Schutz vor einer Wiederverwendung einer Ausgabeschnittstelle (200) nach einem der vorhergehenden Ansprüche, wobei das Verfahren das Folgende umfasst:
Spreizen, mittels des Spreizers (500, 600), der Klingen (301) der Aufnahmeöffnung (300), wenn sich die Aufnahmeöffnung (300) im gespreizten Zustand befindet, so dass der Öffnungsdurchmesser (305) der Öffnung (304) zumindest so groß wie der Außendurchmesser (43) des Verbindungsteils (42) der medizinischen Vorrichtung (10) ist;
Aufnehmen, wenn sich die Aufnahmeöffnung (300) im gespreizten Zustand befindet, des Verbindungsteils (42) der medizinischen Vorrichtung (10) in der Aufnahmeöffnung (300);
Bewegen, wenn das Verbindungsteil (42) der medizinischen Vorrichtung (10) aufgenommen wird, des Spreizers (500, 600) aus einer ersten Position in der Aufnahmeöffnung (300) in eine zweite Position in der Aufnahmeöffnung (300);
Verriegeln, wenn sich der Spreizer (500, 600) in der zweiten Position befindet, des Spreizers (500, 600) an der Basis der Aufnahmeöffnung (300); und
Blockieren, wenn sich die Aufnahmeöffnung (300) im geschlossenen Zustand befindet, der Aufnahme des Verbindungsteils (42) der medizinischen Vorrichtung (10) .

## Revendications

1. Interface de distribution (200) pouvant être fixée à un dispositif médical (10),
l'interface de distribution (200) comprenant :
une ouverture de réception (300) comprenant des lamelles (301) agencées pour former une ouverture (304) et pour être resserrées dans la direction du centre de l'ouverture (304) par une force élastique,
l'ouverture de réception (300) ayant un état fermé et un état déployé,
dans l'état fermé, l'ouverture (304) ayant un diamètre d'ouverture (305) qui est plus petit qu'un diamètre externe (43) d'une partie de raccordement (42) du dispositif médical (10), et
dans l'état déployé, le diamètre d'ouverture (305) de l'ouverture (304) étant au moins aussi grand que le diamètre externe (43) de la partie de raccordement (42) du dispositif médical (10), et
l'ouverture de réception (300) étant conçue pour recevoir la partie de raccordement (42) du dispositif médical (10) dans l'état déployé et pour bloquer la réception de ladite partie de raccordement (42) dudit dispositif médical (10) dans l'état fermé ; et
un élément de déploiement (500, 600) qui est au moins partiellement conique et peut être inséré dans l'ouverture de réception (300) et
l'élément de déploiement (500, 600) est conçu, pendant l'introduction, pour se déplacer dans l'ouverture de réception (300) de sorte que les lamelles (301) glissent le long de la surface au moins partiellement conique de l'élément de déploiement (500, 600) pour déployer les lamelles (301) de ladite ouverture de réception (300) et agrandir un diamètre d'ouverture (305) de l'ouverture de réception (300), lorsque l'ouverture de réception (300) est dans l'état déployé.

2. Interface de distribution selon la revendication 1, dans laquelle ledit élément de déploiement (500, 600) est en outre conçu pour permettre que les lamelles (301) de ladite ouverture de réception (300) se ferment dans une direction d'un centre de l'ouverture (304) lorsque ladite ouverture de réception (300) est dans l'état fermé.

3. Interface de distribution selon l'une quelconque des revendications 1 et 2, ledit appareil comprenant en outre :
un raccord (244) conçu pour se raccorder à ladite partie de raccordement (42) dudit dispositif médical (10), ledit raccord (244) étant agencé dans ladite ouverture de réception (300).

4. Interface de distribution selon la revendication 3, dans laquelle les lamelles (301) sont agencées cylindriques et/ou coniques autour dudit raccord (244).

5. Interface de distribution selon l'une quelconque des revendications 1 à 4, dans laquelle ledit élément de déploiement (500, 600) est conçu pour se déplacer depuis une première position dans ladite ouverture de réception (300) dudit appareil vers une seconde position dans ladite ouverture de réception (300) dudit appareil.

6. Interface de distribution selon la revendication 5, dans laquelle, lorsque l'élément de déploiement (500, 600) est dans la seconde position, ladite ouverture de réception (300) est conçue pour verrouiller ledit élément de déploiement (500, 600) au niveau de la base de l'ouverture de réception (300).

7. Interface de distribution selon l'une quelconque des revendications 1 à 6, dans laquelle ladite ouverture de réception (300) est à ressort.

8. Interface de distribution selon l'une quelconque des revendications 1 à 7, dans laquelle ladite ouverture de réception (300) a une coupe transversale arrondie.

9. Interface de distribution selon l'une quelconque des revendications 1 à 8, dans laquelle ledit élément de déploiement (500, 600) est arrondi et/ou annulaire.

10. Interface de distribution selon l'une quelconque des revendications 1 à 9, dans laquelle un diamètre externe (504) dudit élément de déploiement (500, 600) est au moins aussi grand que ledit diamètre externe (43) de ladite partie de raccordement (42) dudit dispositif médical (10).

11. Interface de distribution selon l'une quelconque des revendications 1 à 10, dans laquelle ladite ouverture de réception (300) et/ou ledit élément de déploiement (500, 600) sont en plastique.

12. Interface de distribution selon l'une quelconque des revendications 1 à 11, dans laquelle ledit dispositif médical (10) est conçu pour éjecter un médicament.

13. Procédé de protection contre une réutilisation pour une interface de distribution (200) selon l'une quelconque des revendications précédentes, le procédé consistant à :
déployer, par l'élément de déploiement (500, 600), les lamelles (301) de l'ouverture de réception (300) lorsque l'ouverture de réception (300) est dans l'état déployé, de sorte que le diamètre d'ouverture (305) de l'ouverture (304) soit au moins aussi grand que le diamètre externe (43) de la partie de raccordement (42) du dispositif médical (10) ;
recevoir, lorsque l'ouverture de réception (300) est dans l'état déployé, dans ladite ouverture de réception (300), la partie de raccordement (42) du dispositif médical (10) ;
déplacer, lors de la réception de ladite partie de raccordement (42) dudit dispositif médical (10), ledit élément de déploiement (500, 600) depuis une première position dans ladite ouverture de réception (300) vers une seconde position dans ladite ouverture de réception (300) ;
verrouiller, lorsque l'élément de déploiement (500, 600) est dans la seconde position, ledit élément de déploiement (500, 600) au niveau de la base de l'ouverture de réception (300) ; et
bloquer, lorsque l'ouverture de réception (300) est dans l'état fermé, ladite réception de ladite partie de raccordement (42) dudit dispositif médical (10).
